# EUROPÄISCHE PATENTANMELDUNG

(11) **EP 2 135 641 A1**
(43) Veröffentlichungstag der Anmeldung: **23.12.2009**
(21) Anmeldenummer: 09159863.1
(22) Anmeldetag: 11.05.2009
(51) Int. Cl.: A61N 1/39

(54) **Antitachykarder Herzstimulator**

(30) Priorität: 09.06.2008 DE 102008002293
(71) Anmelder: Biotronik CRM Patent AG, 6341 Baar (CH)
(72) Erfinder: Dörr, Thomas, 12437, Berlin (DE)
(74) Vertreter: Lindner-Vogt, Karin L.

(57) **Zusammenfassung**

Die Erfindung betrifft einen implantierbaren Herzstimulator mit einer Kardioversions-/Defibrillationseinheit, die mit mindestens einem Elektrodenpaar zur Defibrillations-/Kardioversionsschocktherapie verbunden oder zu verbinden und ausgebildet ist, Kardioversionsschocks oder Defibrillationsschocks zu generieren und abzugeben, einer atrialen Sensingeinheit, die ausgebildet ist, eine Vorhofkontraktion zu erfassen und im Falle einer detektierten Vorhofkontraktion ein ein jeweiliges atriales Ereignis anzeigendes atriales Sensingsignal auszugeben, einer ventrikulären Sensingeinheit, die ausgebildet ist, eine jeweilige Kammerkontraktion zu erfassen und im Falle einer detektierten Kammerkontraktion ein ventrikuläres Sensingsignal auszugeben, einer Tachykardieerfassungseinheit, die mit der atrialen und der ventrikulären Sensingeinheit verbunden und ausgebildet ist, eine Tachykardie zu erfassen und als ventrikuläre Tachykardie (VT) oder als superventrikuläre Tachykardie (SVT) zu klassifizieren, und einer Therapiesteuereinheit, die zum Auslösen eines atrialen Kardioversionsschocks mit der Kardioversions/Defibrillationseinheit verbunden und ausgebildet ist, mindestens einen atrialen Kardioversionsschock auszulösen, wenn zunächst ein von der ventrikulären Sensingeinheit erfasster ventrikulärer Rhythmus schneller als eine programmierte Frequenzgrenze ist und gleichzeitig Tachykardieerfassungseinheit eine SVT als Vorhofflimmern (AFib) klassifiziert.

## Beschreibung

Die Erfindung betrifft einen implantierbaren Herzstimulator für die Therapie tachykarder Herzrhythmusstörungen. Ein solcher Herzstimulator ist auch als implantierbarer Cardioverter/Defibrillator (ICD) bekannt und erfüllt in der Regel auch die Funktion eines implantierbaren Herzschrittmachers.

Tachykarde Herzrhythmusstörungen können einerseits Tachykardien im engeren Sinne sein, bei denen eine jeweils betroffene Herzkammer (Ventrikel oder Atrium) zwar geordnet, aber mit einer physiologisch unangemessen hohen Rate kontrahiert. Bei einer Fibrillation (Flimmern) kommt es dagegen zu einer ungeordneten Kontraktion der jeweiligen Herzkammer infolge kreisender Erregung, bei der die jeweils betroffene Herzkammer praktisch nicht mehr zur Förderung eines Blutvolumens beiträgt.

Tachykardien können nach dem Ort ihrer Entstehung unterschieden werden. Ventrikuläre Tachykardien haben ihren Ursprung im Ventrikel, während superventrikuläre Tachykardien (SVT) ihren Ursprung oberhalb des Ventrikels, also beispielsweise im Atrium, haben.

Implantierbare Herzstimulatoren in Form von Herzschrittmachern oder Cardiovertern/Defibrillatoren sind grundsätzlich bekannt. Solche Herzstimulatoren sind in der Regel an Elektrodenleitungen angeschlossen, die in einer Kammer eines Herzens oder in unmittelbarer Nähe Stimulations- und optional zusätzliche Defibrillationselektroden besitzen. Über eine Stimulationselektrode kann ein Herzschrittmacher einen elektrischen Stimulationsimpuls an das Muskelgewebe einer Herzkammer abgeben, um so eine stimulierte Kontraktion der Herzkammer hervorzurufen, sofern der Stimulationsimpuls eine ausreichende Intensität besitzt und das Herzmuskelgewebe (Myokard) sich nicht gerade in einer refraktären Phase befindet. Um auf diese Weise eine stimulierte Kontraktion einer Herzkammer auszulösen, werden üblicher Weise Elektrodenleitungen mit relativ kleinflächigen Stimulationselektroden verwendet, da es zum Auslösen einer stimulierten Kontraktion einer Herzkammer ausreicht, wenn nur ein kleiner Teil des Myokards dieser Herzkammer anfänglich stimuliert wird. Eine derartige stimulierte Kontraktion einer Herzkammer wird im Rahmen dieser Beschreibung als stimuliertes Ereignis bezeichnet. Kommt es zu einer natürlichen Kontraktion der Herzkammer, wird dies im Rahmen dieser Beschreibung als Eigenaktion oder als intrinsisches Ereignis bezeichnet. Eine Kontraktion beispielsweise des rechten Atriums eines Herzens wird als atriales Ereignis bezeichnet, welches beispielsweise ein natürliches atriales Ereignis sein kann, oder - im Falle eines atrialen Herzschrittmachers - auch ein stimuliertes atriales Ereignis. Im gleichen Sinne können natürliche (intrinsische) und stimulierte linksventrikuläre und rechtsventrikuläre Ereignisse unterschieden werden.

Eine lokale Erregung des Myokards breitet sich vom Erregungsort ausgehend per Reizleitung im Myokard aus und führt zu einer Depolarisation der Muskelzellen und damit zu einer Kontraktion des Myokards. Nach kurzer Zeit kommt es zu einer Repolarisation der Muskelzellen und damit zu einer Entspannung des Myokards. Während der Phase der Depolarisation sind die Herzmuskelzellen für eine Erregung unempfindlich, d.h. refraktär. Die mit der Depolarisation und Repolarisation einhergehenden elektrischen Potentiale können wahrgenommen und deren zeitlicher Verlauf - als Elektrokardiogramm bezeichnet - ausgewertet werden.

Das Erfassen solcher natürlichen (intrinsischen) Ereignisse erfolgt durch Ableiten der elektrischen Potenziale des Myokards der jeweiligen Herzkammer mit Hilfe von Sensingelektroden, die Teil einer entsprechenden Elektrodenleitung sind. Dabei können die Sensingelektroden gleichzeitig die Stimulationselektroden sein und abwechselnd als Stimulations- und als Sensingelektroden verwendet werden. Typischerweise ist für das Sensing ein Elektrodenpaar bestehend aus einer Spitzenelektrode (Tip-Elektrode) und einer Ringelektrode vorgesehen, von denen die Spitzenelektrode auch als Stimulationselektrode dient. Auf diese Weise erfolgt eine bipolare Ableitung eines intrakardialen Elektrokardiogramms (IEGM). Dabei erfolgt das Sensing und die Stimulation im Ventrikel mit Hilfe einer ventrikulären Elektrodenleitung und die Stimulation und das Sensing im Atrium (im rechten Atrium) mit einer atrialen Elektrodenleitung, die separat an den jeweiligen Herzstimulator angeschlossen sind. Zusätzlich kann auch eine linksventrikuläre Elektrodenleitung vorgesehen sein, die typischer Weise über den Koronarsinus und eine von diesem abzweigende Lateralvene in die Nähe des linken Ventrikels ragt und dort eine kleinflächige Stimulations- und/oder Sensingelektrode aufweisen kann.
Die Sensingelektroden sind im Betrieb des Herzstimulators mit entsprechenden Sensingeinheiten verbunden, die ausgebildet sind, ein jeweiliges über eine Sensingelektrode (bzw. ein Sensingelektrodenpaar) aufgenommenes Elektrokardiogramm auszuwerten und insbesondere intrinsische atriale bzw. ventrikuläre Ereignisse zu detektieren, d.h. natürliche atriale oder ventrikuläre Kontraktionen. Dies geschieht beispielsweise durch Schwellwertvergleich, d.h. ein intrinsisches Ereignis wird detektiert, wenn ein jeweiliges intrakardiales Elektrokardiogramm einen geeignet vorgegebenen Schwellwert überschreitet.

Aus der Frequenz, mit der atriale bzw. ventrikuläre Ereignisse aufeinander folgen, kann die jeweilige intrinsische atriale Herzrate (atriale Frequenz) bzw. ventrikuläre Herzrate (Ventrikelfrequenz) abgeleitet und somit beispielsweise Tachykardien detektiert werden.

Die derzeit am Markt verfügbaren ICDs bieten eine Reihe von Funktionen zur Terminierung atrialer Arrhythmien, insbesondere atrialen Flimmerns durch automatisch abgegebene Kardioversionsschocks. Diese Kardioversionsschocks werden immer auf eine Vorhofflimmerdetektion hin ausgelöst. Der ventrikuläre Rhythmus wird hier nur zur sicheren Synchronisation des Schocks bewertet.

Alle derzeit bekannten Geräte geben diese Kardioversionsschocks entweder unabhängig von ventrikulären Rhythmus ab oder geben dann keinen Kardioversionsschock ab, wenn die ventrikuläre Frequenz einen programmierbaren Wert übersteigt. In vielen Anwendungen wird die atriale Kardioversion nur zu bestimmten Tages- bzw. Nachtzeiten zugelassen und ggf. zunächst verzögert, um einer spontanen Terminierung einen Vorzug zu geben.

Für die adäquate Therapie von Herzinsuffizienzpatienten mit persistierendem Vorhofflimmern existiert derzeit noch kein hinreichend spezifischer Algorithmus zur Vorhofflimmertherapie. Bei diesen Patienten ist ein Vorhofflimmern immer dann behandlungspflichtig, wenn das Vorhofflimmern schnell in den Ventrikel übergeleitet wird. Diese Patienten verlieren durch das Vorhofflimmern zum einen den ventrikulären Füllungsanteil durch eine optimierte AV-Zeit; übersteigt die ventrikuläre Reaktion eine bestimmte Frequenz, verlieren diese Patienten zusätzlich die ventrikuläre Resynchronisation und mit steigender Ventrikelfrequenz sinkt gleichzeitig das Herzzeitvolumen bei zunehmendem myokardialen Sauerstoffbedarf. Dies führt bei diesen Patienten zur raschen Dekompensation. Einzig effektive Therapie ist hier die sofortige Kardioversion des Vorhofflimmerns, sofern ein Thromboserisiko weitgehend ausgeschlossen werden kann.

Aufgabe der Erfindung ist es, einen Herzstimulator zu schaffen, der eine atriale Tachykardie zu einem geeigneten Zeitpunkt beendet.

Erfindungsgemäß ist hierzu ein implantierbarer Herzstimulator zur elektrischen antitachykarden Therapie des Herzens vorgesehen,
- mit mindestens einer atrialen und einer ventrikulären Sensingeinheit und
- mit mindestens einem Schockgenerator zur Defibrillations-/Kardioversionsschocktherapie,
- mit einer Diskriminierungseinheit, die zur Unterscheidung ventrikulärer (VT) und supraventrikulärer Tachykardien (SVT) ausgebildet ist, sowie
- mit einer Steuereinheit, die zum Auslösen eines atrialen Kardioversionsschocks mit dem Schockgenerator verbunden und derart konfiguriert ist, dass mindestens ein atrialer Kardioversionsschock ausgelöst wird, wenn zunächst der ventrikuläre Rhythmus schneller als eine programmierte Frequenzgrenze ist und gleichzeitig die Diskriminierungseinheit eine SVT als Vorhofflimmern (AFib) klassifiziert.

Die Steuereinheit ist in an sich bekannter Weise in der Lage, mittels von der atrialen und der ventrikulären Sensingeinheit erfasster natürlicher (intrinsischer) atrialer bzw. ventrikulärer Ereignisse einen jeweiligen intrinsischen atrialen und ventrikulären Herzrhythmus mit einer entsprechenden atrialen Frequenz bzw. Ventrikelfrequenz zu erfassen.

Ein erfindungsgemäßer implantierbarer Herzstimulator umfasst somit wenigstens:
- eine Kardioversions-/Defibrillationseinheit, die mit mindestens einem Elektrodenpaar zur Defibrillations-/Kardioversionsschocktherapie verbunden oder zu verbinden und als Schockgenerator ausgebildet ist, Kardioversionsschocks oder Defibrillationsschocks zu generieren und abzugeben,
- eine atriale Sensingeinheit, die ausgebildet ist, eine Vorhofkontraktion zu erfassen und im Falle einer detektierten Vorhofkontraktion ein eine jeweiliges atriales Ereignis anzeigendes atriales Sensingsignal auszugeben,
- eine ventrikuläre Sensingeinheit, die ausgebildet ist eine jeweilige Kammerkontraktion zu erfassen und im Falle einer detektierten Kammerkontraktion ein ventrikuläres Sensingsignal auszugeben,
- eine Tachykardieerfassungseinheit, die mit der atrialen und der ventrikulären Sensingeinheit verbunden und ausgebildet ist, eine Tachykardie zu erfassen, wobei die Tachykardieerfassungseinheit weiter als Diskrimierungseinheit ausgebildet, eine erfasste Tachykardie als ventrikuläre Tachykardie (VT) oder als superventrikuläre Tachykardie (SVT) zu klassifizieren, und
- eine Therapiesteuereinheit, die zum Auslösen eines atrialen Kardioversionsschocks mit der Kardioversions-/Defibrillationseinheit verbunden und ausgebildet ist, mindestens einen atrialen Kardioversionsschock auszulösen, wenn zunächst ein von der ventrikulären Sensingeinheit erfasster ventrikulärer Rhythmus schneller als eine programmierte Frequenzgrenze ist und gleichzeitig Tachykardieerfassungseinheit eine SVT als Vorhofflimmern (AFib) klassifiziert.

Der Erfindung liegt der Gedanke zugrunde, ein Vorhofflimmern bei Patienten mit chronischer Herzinsuffizienz immer dann sofort zu terminieren, wenn die resultierende Ventrikelfrequenz einen Grenzwert übersteigt. Dieser Grenzwert ist dabei patientenindividuell und muss vom Arzt festgelegt werden können. Bei dieser Kardioversion ist das Risiko eines Schlaganfalls (Ablösung eines Thrombus im Systemkreislauf) möglichst zu minimieren.

Vorzugsweise ist die Steuereinheit ausgebildet, eine atriale Kardioversion immer nur dann auszulösen, wenn der ventrikuläre Rhythmus schneller als eine programmierbare Frequenzgrenze jedoch langsamer als der atriale Rhythmus ist und der ventrikuläre Rhythmus unregelmäßig ist, d.h. ein vorgegebenes Stabilitätskriterium nicht erfüllt.

Außerdem ist ein Herzstimulator bevorzugt, dessen Therapiesteuereinheit ausgebildet ist, eine atriale Kardioversion immer auf eine R-Welle, d.h. ein detektiertes intrinsisches Ereignis im Ventrikel zu synchronisieren.

Die Kardioversions-/Defibrillationseinheit ist vorzugsweise mit mindestens drei Schockelektroden, nämlich einer distalen Schockwendel, einer proximalen Schockwendel an der rechtsventrikulären Elektrodenleitung und einem elektrisch leitenden Gehäuse des implantierbaren Herzstimulators verbunden oder zu verbinden.

Darüber hinaus ist die Kardioversions-/Defibrillationseinheit in Verbindung mit der Therapiesteuereinheit vorzugsweise so ausgebildet, dass die Kardioversions-/Defibrillationseinheit für eine atriale Kardioversion und für eine ventrikuläre Defibrillation mit jeweils wenigstens zwei Schockelektroden verbunden ist, von den wenigstens eine Schockelektrode für die atriale Kardioversion von den Schockelektroden für die ventrikuläre Defibrillation verschieden ist, so dass für die atriale Kardioversion ein anderer Schockpfad eingestellt werden kann als für die ventrikuläre Defibrillation. Beispielsweise kann die Kardioversions-/Defibrillationseinheit für die atriale Kardioversion mit der proximalen Schockwendel der rechtsventrikulären Elektrodenleitung und dem Gehäuse des implantierbaren Herzstimulators verbunden sein und für die ventrikuläre Defibrillation mit der distalen Schockwendel der rechtsventrikulären Elektrodenleitung und dem Gehäuse des implantierbaren Herzstimulators.

Weiterhin ist der implantierbare Herzstimulator vorzugsweise ein biventrikulärer Dreikammer Herzschrittmacher, der mit einer atrialen und zwei ventrikulären Elektrodenleitungen verbunden (CRT) bzw. zu verbinden ist, nämlich einer rechtsatrialen Elektrodenleitung, einer rechtsventrikulären Elektrodenleitung und einer linksventrikulären Elektrodenleitung. Ein solcher Herzstimulator kann für die kardiale Resynchronisationstherapie eingesetzt werden und besitzt eine linksventrikuläre Sensingeinheit, die mit einer Sensingelektrode an der linksventrikulären Elektrodenleitung verbunden oder zu verbinden ist und ausgebildet ist, linksventrikuläre intrinsische Ereignisse zu erfassen.

Bei einem derartigen Herzstimulator ist die Therapiesteuereinheit vorzugsweise mit der atrialen und der linksventrikulären Sensingeinheit verbunden.

Vorzugsweise ist die Therapiesteuereinheit weiter ausgebildet, eine Synchronisation der atrialen Kardioversion immer auf eine linksventrikulär wahrgenommene R-Welle vorzunehmen.

Vorzugsweise weist der implantierbare Herzstimulator einen Zeitgeber auf, der mit der atrialen Sensingeinheit verbunden ist und ausgebildet ist, zu überwachen, ob ein Vorhofflimmern ununterbrochen eine vorgegebene Zeitdauer übersteigt. Wird diese Zeit überschritten, dann wird die atriale Kardioversion gesperrt.

Vorzugsweise ist die Therapiesteuereinheit derart programmierbar, dass die Auslösung einer automatischen atrialen Kardioversion durch einen programmierbaren Parameter ein- bzw. ausgeschaltet werden kann. Hierzu ist der implantierbare Herzstimulator vorzugsweise mit einer Telemetrieeinheit und einem Speicher für Programmparameter ausgestattet, so dass der implantierbare Herzstimulator von außen fernprogrammierbar ist.

Die Erfindung soll nun anhand eines Ausführungsbeispiels mit Bezug auf die Figuren näher erläutert werden. Von den Figuren zeigen
- Figur 1:: einen implantierbaren Herzstimulators in Form eines Zweikammer-Herzschrittmachers in Verbindung mit einem Patientengerät;
- Figur 2:: ein Übersichtsschaltbild des implantierbaren Herzstimulators;
- Figur 3:: ein detaillierteres Blockschaltbild einer erweiterten Diskriminierungs-Einheit zur atrialen Kardioversion des implantierbaren Herzstimulators;
- Figur 4:: ein Diagramm zur Erläuterung der Funktionsweise der Diskrimierungseinheit bei der Klassifikation tachykarder Arrhythmien;
- Figur 5:: eine alternative Ausführungsform eines Herzstimulators in Form eines biventrikulären Dreikammer-Herzschrittmachers;
- Figur 6:: eine Illustration eines Schockpfades für eine ventrikuläre Defibrillation; und
- Figur 7:: eine Illustration eines Schockpfades für eine atriale Kardioversion.

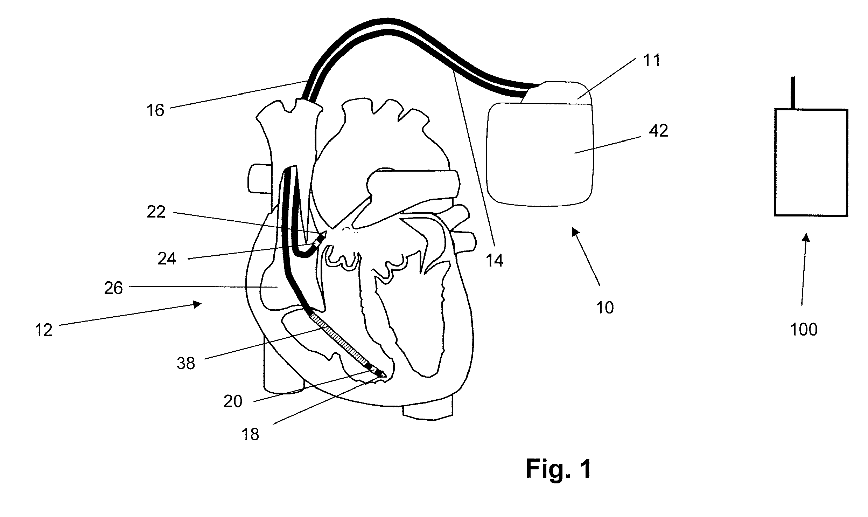

Figur 1 zeigt einen Zweikammer-Herzschrittmacher und implantierbaren Kardioverter/Defibrillator (ICD) als implantierbaren Herzstimulator 10. Dieser Zweikammer-Herzschrittmacher ist über Elektrodenleitungen 14 und 16 mit Stimulations- und Sensingelektroden 18 und 20 beziehungsweise 22 und 24 im Ventrikel beziehungsweise Atrium eines Herzens verbunden und kann auf diese Weise Stimulationsimpulse an das Herz abgeben und elektrische Potentiale vom Herzen aufnehmen.

Außerdem ist ein externes Gerät 100 in der Nähe des implantierbaren Herzstimulators 10 dargestellt.

Die Elektrodenleitungen 14 und 16 sind über bekannte, standardisierte Steckverbindungen mit Kontaktbuchsen in einem Header (Anschlussgehäuse) 11 des Herzstimulators 10 elektrisch verbunden. Auf diese Weise sind die Elektrodenleitungen 14 und 16 auch mit elektronischen Komponenten im Inneren eines hermetisch dichten Metallgehäuses 42 des Herzstimulators 10 verbunden. Diese Komponenten sind nachfolgend detaillierter schematisch dargestellt und bestimmen die erfindungsgemäße Funktionsweise des Herzstimulators 10.

Die Elektrodenleitung 14 ist eine rechtsatriale Elektrodenleitung und besitzt an ihrem distalen Ende eine atriale Spitzenelektrode RA Tip 22 sowie in kurzer Entfernung davon eine atriale Ringelektrode RA Ring 24, die beide im rechten Atrium 26 des Herzens 12 platziert sind.

Die Elektrodenleitung 16 ist eine rechtsventrikuläre Elektrodenleitung und besitzt an ihrem distalen Ende eine rechtsventrikuläre Spitzenelektrode RV Tip 18 und in unmittelbarer Nähe eine rechtsventrikuläre Ringelektrode RV Ring 20. Beide Elektroden sind im Apex des rechten Ventrikels des Herzens angeordnet.

Außerdem trägt die rechtsventrikuläre Elektrodenleitung 16 noch eine rechtsventrikuläre Schockwendel RV Coil 38 als großflächige Elektrode zur Abgabe von Defibrillationsschocks.

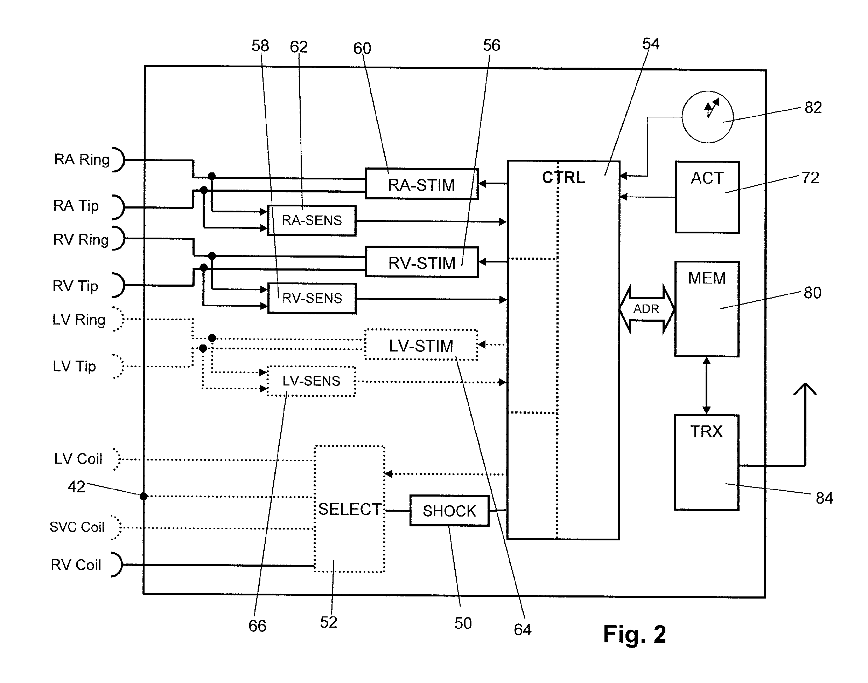

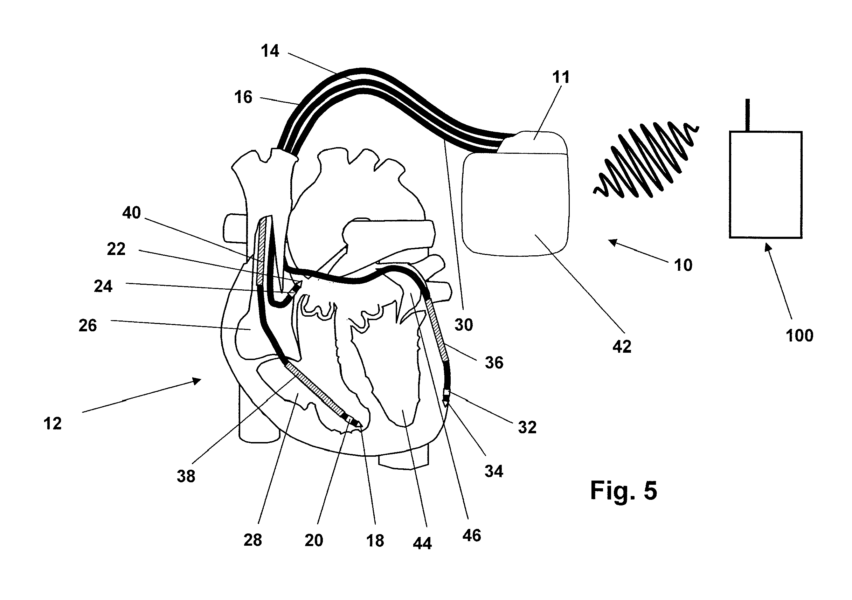

In Figur 2 sind die Hauptbestandteile des Herzstimulators 10 dargestellt. In punktierter Liniendarstellung sind außerdem weitere Komponenten dargestellt, wie sie bei einer alternativen Ausführungsvariante eines implantierbaren Herzstimulators 10' (siehe Figur 5) zusätzlich vorgesehen sein können.

Auf der linken Seite sind die elektrischen Anschlüsse für die verschiedenen Elektroden 18, 20, 22, 24 und 38 dargestellt. Die Schockelektrode (Schockwendel) 38 ist mit einem Schockimpulsgenerator 50 verbunden. Der Schockgenerator 50 ist mit einer Stimulationssteuereinheit 54 verbunden, die den Schockimpulsgenerator 50 bei Bedarf zur Erzeugung und Abgabe eines Kardiversions- oder Defibrillationsschocks ansteuert.

Der Anschluss für die rechtsventrikuläre Spitzenelektrode RV Tip 18 sowie der Anschluss für die rechtsventrikuläre Ringelektrode RV Ring 20 sind jeweils sowohl mit einer rechtsventrikulären Stimulationseinheit 56 als auch mit einer rechtsventrikulären Sensingeinheit 58 verbunden. Sowohl die rechtsventrikuläre Stimulationseinheit 56 als auch die rechtsventrikuläre Sensingeinheit 58 sind jeweils mit der Stimulationssteuereinheit 54 verbunden.

Die rechtsventrikuläre Stimulationseinheit 56 ist dazu ausgebildet, auf ein Ansteuersignal der Stimulationssteuereinheit 54 hin einen rechtsventrikulären Stimulationsimpuls zu erzeugen und im Anschluss an die rechtsventrikuläre Ringelektrode RV Ring 20 und die rechtsventrikuläre Spitzenelektrode RV Tip 18 abzugeben. Alternativ ist es auch möglich, dass das Gehäuse 42 des Herzstimulators 10 eine neutrale Elektrode bildet und die rechtsventrikuläre Stimulationseinheit 56 mit dem Anschluss für die rechtsventrikuläre Spitzenelektrode RV Tip 18 und dem Gehäuse 42 als andere Elektrode zur Abgabe eines Stimulationsimpulses verbunden ist. Ein rechtsventrikulärer Stimulationsimpuls unterscheidet sich von einem Defibrillationsschock dadurch, dass der Stimulationsimpuls eine wesentlich geringere Impulsstärke besitzt, sodass er nicht wie ein Defibrillationsschock auf einen Schlag das vollständige Herzgewebe (Myokard) einer Herzkammer erregt, sondern nur die Herzmuskelzellen in unmittelbarer Umgebung der rechtsventrikulären Spitzenelektrode RV Tip 18. Diese Erregung breitet sich dann durch natürliche Reizleitung über den gesamten Ventrikel weiter aus und sorgt so für eine stimulierte Kontraktion des Ventrikels.

Die rechtsventrikuläre Sensingeinheit 58 ist dazu ausgebildet, an dem Anschluss für die rechtsventrikuläre Ringelektrode RV Ring 20 und die rechtsventrikuläre Spitzenelektrode RV Tip 18 anliegende elektrische Potentiale zunächst durch einen Eingangsverstärker zu verstärken und zu filtern. Weiterhin ist die rechtsventrikuläre Sensingeinheit ausgebildet, den Verlauf der an ihren Eingängen anliegenden elektrischen Signale derart auszuwerten, dass die rechtsventrikuläre Sensingeinheit 58 selbsttätig eine natürliche (intrinsische), d. h. selbsttätige Kontraktion des rechten Ventrikels detektiert. Dies kann beispielsweise dadurch geschehen, dass der Verlauf des an den Eingängen der rechtsventrikulären Sensingeinheit 58 anliegenden Signals mit einem Schwellwert verglichen wird. Typischerweise ist die größte Amplitude des Signals in Form der so genannten R-Zacke kennzeichnend für eine natürliche Kontraktion des rechten Ventrikels, die durch Schwellwertvergleich detektiert werden kann. Die rechtsventrikuläre Sensingeinheit 58 gibt daraufhin ein entsprechendes, eine natürliche Kontraktion des rechten Ventrikels anzeigendes Ausgangssignal an die Stimulationssteuereinheit 54 aus.

In analoger Weise sind der Anschluss für die rechtsatriale Spitzenelektrode RA Tip 22 und der Anschluss für die rechtsatriale Ringelektrode RA Ring 24 sowohl mit einer rechtsatrialen Stimulationseinheit 60 als auch mit einer rechtsatrialen Sensingeinheit 62 verbunden, die jeweils ihrerseits wiederum mit der Stimulationssteuereinheit 54 verbunden sind. Die rechtsatriale Stimulationseinheit 60 ist dazu ausgebildet, Stimulationsimpulse zu erzeugen, deren Stärke ausreicht, das rechtsatriale Myokard zu erregen. Die rechtsatrialen Stimulationsimpulse können dabei eine andere Impulsstärke besitzen, als die rechtsventrikulären Stimulationsimpulse. Die rechtsatriale Sensingeinheit 62 ist ausgebildet, aus dem Verlauf des an ihren Eingängen anliegenden Differenzsignals eine sogenannte P-Welle zu detektieren, die eine natürliche (intrinsische) Kontraktion des rechten Atriums kennzeichnet. Detektiert die rechtsatriale Sensingeinheit 62 eine entsprechende P-Welle, erzeugt sie ein Ausgangssignal und gibt dieses an die Stimulationssteuereinheit 54 weiter, welches eine natürliche Kontraktion des rechten Atriums kennzeichnet.

Als weiterer Bestandteil des Herzstimulators 10 ist ein Aktivitätssensor 72 mit der Stimulationssteuereinheit 54 verbunden. Der Aktivitätssensor 72 ist dazu ausgebildet, ein von der körperlichen Aktivität eines Patienten abhängiges Signal, zum Beispiel ein Bewegungssignal, zu erfassen und ein entsprechendes, die körperliche Aktivität des Patienten anzeigendes Signal an die Stimulationssteuereinheit 54 auszugeben. Dies erlaubt es, dass die Stimulationssteuereinheit 54 das Timing der Stimulationsimpulse an den Bedarf des Patienten (hämodynamischen Bedarf) anpasst.

Weiterhin umfasst der Herzstimulator 10 eine Speichereinheit 80, die mit der Stimulationssteuereinheit 54 verbunden ist und es erlaubt, von der Stimulationssteuereinheit 54 erzeugte oder ausgewertete Signale zu speichern. Andererseits erlaubt es die Speichereinheit 80, Steuerprogramme für die Stimulationssteuereinheit 54 in veränderbarer Form zu speichern. Des Weiteren ist die Stimulationssteuereinheit 54 mit einem Zeitgeber 84 verbunden.

Der Herzstimulator 10 verfügt über mindestens eine bidirektionale Telemetrieschnittstelle 84, um gespeicherte Daten vom elektronischen Implantat an ein externes Gerät 100 übertragen zu können und umgekehrt auch Programmiereinstellungen und Therapiekommandos von diesem externen Gerät 100 empfangen zu können.

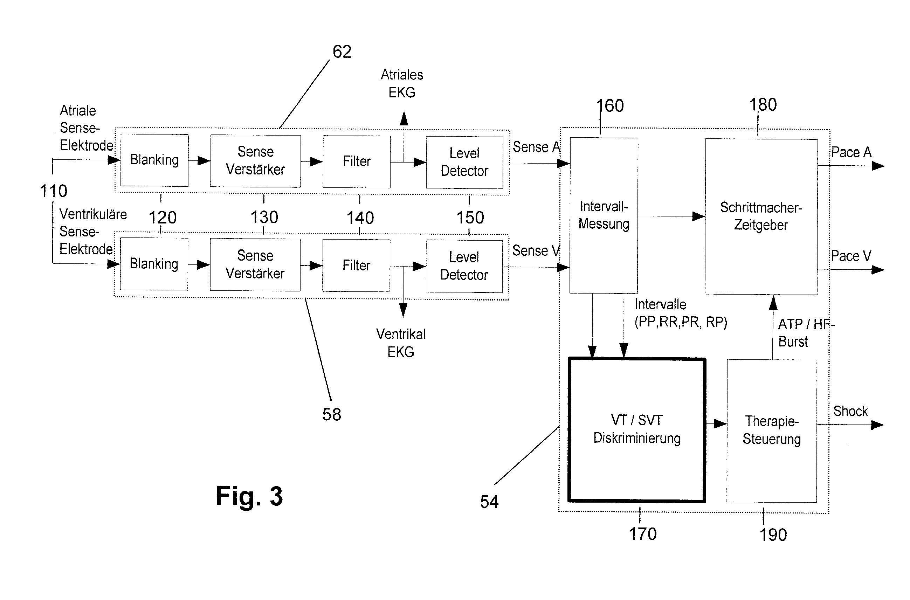

In Figur 3 ist ein Ausschnitt eines detaillierteren Blockschaltbilds eines Zweikammer-ICD, einschließlich der erweiterten Diskriminierungs-Einheit 170 zur atrialen Kardioversion dargestellt.

Figur 3 zeigt die atriale Sensingeinheit 62 und die rechtsventrikuläre Sensingeinheit 58 (oder - falls vorhanden (siehe Figur 5) - die bevorzugte linksventrikuläre Sensingeinheit 66) in detaillierter Darstellung: Die intrakardialen Signale, abgeleitet mittels atrialer bzw. ventrikulärer Sensingelektroden 110, werden zunächst verstärkt, wobei jedem Verstärker 130 jeweils eine Blankingstufe 120 zur Ausblendung von Stimulationsartefakten vorgeschaltet ist. Anschließend wird das jeweilige Signal digitalisiert und gefiltert (Filter 140) und einem Komparator 150 zur P- bzw. R-Wellenwahrnehmung weitergeleitet.

Anschließend erfolgt die weitere Signalverarbeitung in der Steuereinheit 54.

Die mittels der atrialen Sensingeinheit 62 bzw. der rechtsventrikulären Sensingeinheit 58 gewonnen P- und R-Wellen synchronen Ereignisse werden in einer Intervallmesseinheit 160 vermessen. Diese PP-, PR-, RP- und RR-Intervalle werden einem Schrittmacherzeitgeber 180 zur Steuerung der antibradykarden Stimulation weitergeleitet. Ferner werden diese Intervallinformationen in der als Intervallauswerteeinheit dienenden VT/SVT-Diskriminierungseinheit 170 zur Diskriminierung zwischen VT und SVT hinsichtlich ihrer Verhältnisse bezüglich atrialem und ventrikulärem Rhythmus verglichen und in bewerteter Form der Detektions- und Therapiesteuereinheit 190 für die antitachykarde Therapie (HF-Burst, ATP, Schock) verfügbar gemacht.

Die VT/SVT-Diskriminierungseinheit 170 ist derart erweitert, dass ein atrialer Kardioversionsschock immer dann von der Therapiesteuereinheit 190 angefordert wird, wenn ein tachykarder ventrikulärer Rhythmus, bedingt durch ein Vorhofflimmern klassifiziert wird.

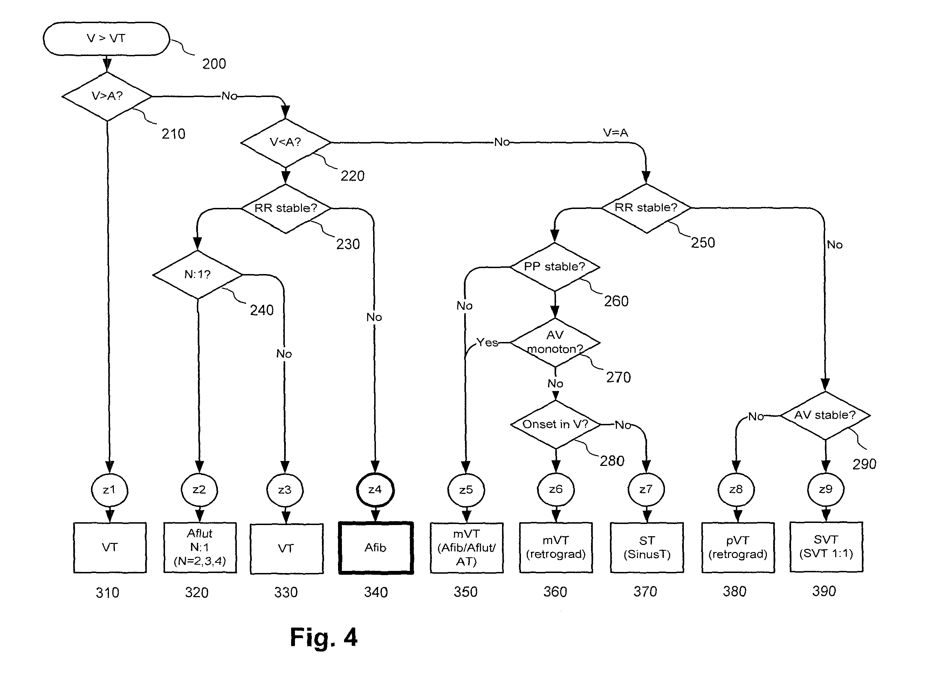

In Figur 4 ist ein Entscheidungsbaum des VT/SVT-Klassifizierers der VT/SVT-Diskriminierungseinheit 170 im Detail dargestellt.

Immer dann, wenn ein ventrikuläres Ereignis (V) schneller als eine programmierte Tachykardiefrequenz wahrgenommen wird (200), wird zunächst geprüft, ob das aktuelle Ventrikelereignis schneller als das aktuelle atriale Ereignis (A) ist (210). Ist V schneller als A, dann erfolgt die Klassifikation als ventrikuläre Tachykardie (310: VT) und der entsprechende Detektionszähler (z1) wird inkrementiert.

Ist V langsamer als A (220), dann wird geprüft, ob die RR-Intervalle stabil sind (230). Bei stabilen RR-Intervallen erfolgt eine Prüfung auf ein festes N:1-Überleitungsverhalten (240). Ist dies gegeben, dann wird ein atriales Flattern (320:AFlut) klassifiziert und der SVT-Zähler z2 inkrementiert.

Ist keine feste Überleitung nachweisbar, dann wird eine VT (330) klassifiziert und z3 inkrementiert.

Werden die RR-Intervalle als instabil bewertet (230), dann wird als Ursache für die schnelle ventrikuläre Frequenz ein Vorhofflimmern (AFib) klassifiziert und der Detektionszähler z4 inkrementiert (340). Dieser Detektionszähler wird für die Kardioversionsentscheidung genutzt.

Sind die atriale und die ventrikuläre Frequenz identisch (V=A), dann wird geprüft, ob die RR-Intervalle stabil sind (250). Bei stabilen RR-Intervallen werden anschließend die PP-Stabilität (260) und die AV-Monotonie (270) geprüft. Sind die PP-Intervalle instabil oder die AV-Monotonie erfüllt, dann wird die ventrikuläre Tachykardie als monomorphe VT bei gleichzeitigem Vorliegen einer atrialen Tachyarrhythmie (350: mVT mit AFib, AFlut oder AT) klassifiziert und der Detektionszähler z5 inkrementiert. Sind die PP-Intervalle hingegen stabil und die AV-Monotonie nicht erfüllt, so wird die Tachykardie dann als retrograd übergeleitete VT (mVT retrograd) klassifiziert, wenn das ventrikuläre Onset-Kriterium (180) erfüllt ist. Der Detektionszähler z6 wird dann inkrementiert.

Ist das Onset-Kriterium (280) nicht erfüllt, wird eine Sinustachykardie (370: SinT) klassifiziert und der Detektionszähler z7 inkrementiert. Wird bei der RR-Stabilitätsprüfung (250) ein instabiler ventrikulärer Rhythmus nachgewiesen, dann entscheidet die Prüfung der AV-Intervallstabilität (290) über eine Klassifikation als retrograd übergeleitete polymorphe VT (280: pVT) in Falle von instabilen AV-Intervallen. Der Detektionszähler z8 wird dann inkrementiert. Sind die AV-Intervalle hingegen stabil, wird eine SVT mit antegrader 1:1-Überleitung klassifiziert (390: SVT 1:1) und der Detektionszähler z9 inkrementiert.

Die in Figur 3 dargestellte VT/SVT-Diskriminierungseinheit 170 entscheidet anhand der in Figur 4 dargestellten Detektionszählerstände (310...390) bei Erreichen eines einstellbaren Zählerstandes über eine Therapieabgabe und triggert dementsprechend die Therapiesteuereinheit 190.

Für die in der Erfindung beschrieben automatische atriale Kardioversion wird der in Figur 4 dargestellte Detektionszähler z4 ausgewertet. Übersteigt der Zählerstand einen vordefinierten Wert, dann gilt die klassifizierte Tachyarrhythmie als Vorhofflimmern mit tachykarder Überleitung in den Ventrikel. Ist in diesem Fall der Parameter: Automatische Kardioversion aktiviert, dann wird von der Detektionseinheit mindestens ein Kardioversionsschock angefordert. Die Schockabgabesteuerung und Synchronisation entspricht dabei einem ventrikulären Defibrillationsschock, jedoch mit dem Zwang, die Schockabgabe auf eine R-Welle zu synchronisieren.

In Figur 5 ist eine alternative Ausführungsvariante eines implantierbaren Herzstimulators in Form eines biventrikulären Dreikammer-Herzstimulators/ICDs 10' dargestellt. Dieser ist über seinen Anschlussblock 11 (Header) mit einer rechtsventrikulären 16, einer rechtsatrialen 14 und zusätzlich einer linksventrikulären Elektrodenleitung 30 verbunden.
Diese Elektrodenleitungen werden im Herzen 12 dauerhaft implantiert. Die rechtsventrikuläre Elektrodenleitung 16 weist dabei am distalen Ende eine bipolare Stimulations- und Wahrnehmungselektrode mit Tip- 18 und Ringelektrode 20 auf. Ferner ist diese Elektrodenleitung mit einer distalen Schockwendel 38 und zusätzlich einer proximalen Schockwendel 40 ausgestattet. Die distale Schockwendel 38 ist dabei so angeordnet, dass diese im rechten Ventrikel 28 liegt. Die proximale Schockwendel 40 liegt im oberen Teil des rechten Atriums 26. Die atriale Elektrodenleitung 14 weist am distalen Ende eine bipolare Stimulations- und Wahrnehmungselektrode, bestehen aus Tip- 22 und Ringelektrode 24 auf, implantiert im rechten Atrium 26. Die linksventrikuläre Elektrodenleitung 30 weist am distalen Ende eine bipolare Stimulations- und Wahrnehmungselektrode mit Tip- 34 und Ringelektrode 32 auf. Ferner ist diese Elektrodenleitung mit einer Schockwendel 36 ausgestattet.

Diese Schockwendel 36 ist dabei so angeordnet, dass diese vom linken Ventrikel 44 bis hinauf zum linken Vorhof 46 reicht. Eine weitere Elektrode für die Schockabgabe stellt das elektrisch aktive Gehäuse 42 des Implantates 10 dar.

Die linksventrikuläre Elektrodenleitung 30 wird vom rechten Atrium 26 des Herzens 12 aus über den Koronarsinus in eine von diesem abzweigende Lateralvene geführt und wird deswegen auch als Koronarsinuselektrodenleitung oder CS-Elektrodenleitung bezeichnet.

Wie Figur 2 anhand der punktiert dargestellten Komponenten zu entnehmen ist, sind auch der Anschluss für die linksventrikuläre Spitzenelektrode LV Tip und der Anschluss für die linksventrikuläre Ringelektrode LV Ring mit einer linksventrikulären Stimulationseinheit 64 und einer linksventrikulären Sensingeinheit 66 verbunden. Die linksventrikuläre Stimulationseinheit 64 und die linksventrikuläre Sensingeinheit 66 sind ebenso mit der Stimulationssteuereinheit 54 verbunden. Beide funktionieren ähnlich wie die bereits beschriebenen Stimulationseinheiten 56 und 60 und Sensingeinheiten 58 und 62.

Die linksventrikuläre Schockwendel 36 ist über einen Anschluss LV-COIL und eine Elektrodenauswahleinheit 52 ebenfalls mit dem Schockgenerator 50 verbunden.

Mittels der Elektrodenauswahleinheit 52 kann die Steuereinheit 54 zwei oder mehr Elektroden (einschließlich des leitenden Gehäuses 42) auswählen, über die ein Schock abgegeben werden soll.

Der in Figur 5 gezeigte Herzstimulator 10' bietet dadurch die Möglichkeit, in Abhängigkeit der primären Ursache der ventrikulären Tachykardie den Schockpfad für die atriale Kardioversion und die ventrikuläre Defibrillation getrennt einzustellen.

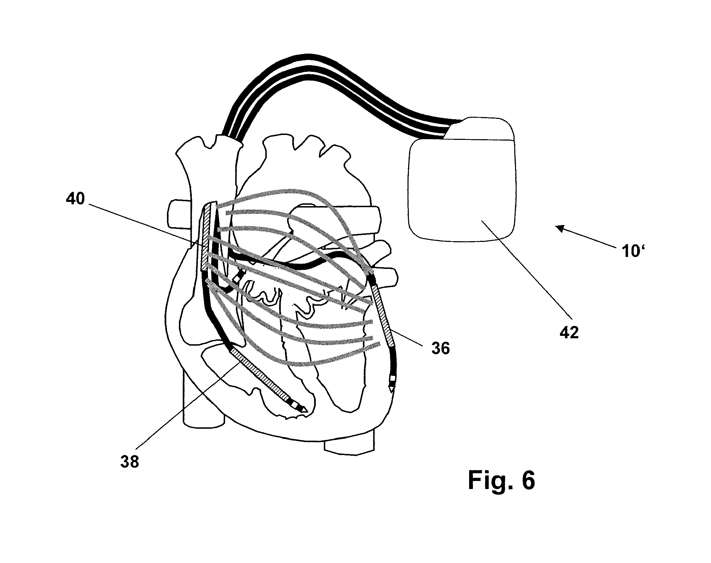

In Figur 6 ist der Schockpfad für die atriale Kardioversion dargestellt. Hier wird die Schockenergie zwischen der rechtsventrikulären proximalen Schockwendel 40 und der linksseitigen Schockwendel 36 abgegeben. So wird bei der Kardioversion die größtmögliche atriale Muskelmasse, insbesondere auch das linke Atrium vom elektrischen Feld erfasst. Die atriale Kardioversion wird immer synchronisiert auf eine Ventrikelerregung synchronisiert, sodass die Gefahr einer ventrikulären Akzeleration minimiert wird.

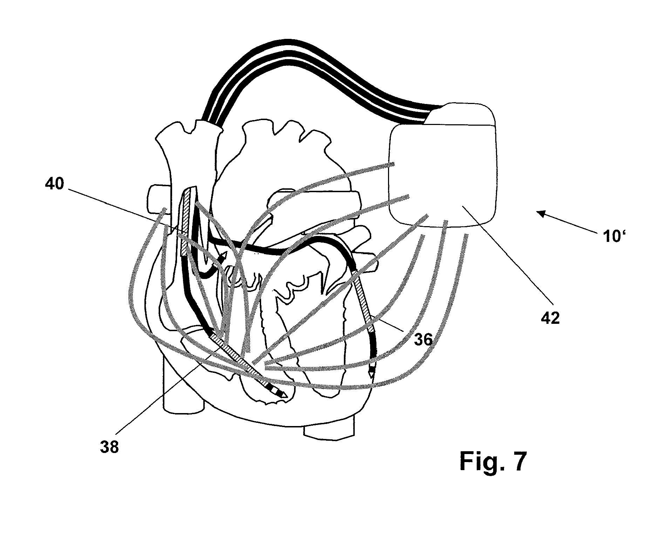

In Figur 7 ist die Strompfadkonfiguration für eine ventrikuläre Defibrillation dargestellt, die z. B. durch den Detektionszähler z3 (330) aus der Figur 4 ausgelöst wird. Hier wird die Schockenergie abgegeben zwischen der distalen rechtsventrikulären Schockwendel 38, der proximalen Schockwendel 40 und dem aktiven Gehäuse 42 des Implantates, wobei das Gehäuse 42 und die proximale Schockwendel 40 im Moment der Schockabgabe auf das gleiche Potential geschaltet werden. So wird bei der Defibrillation eine möglichst große ventrikuläre Muskelmasse erfasst. Diese Schockpfadkonfiguration entspricht der Standardeinstellung der meisten ventrikulären ICDs.

Um das Schlaganfallrisiko, ausgelöst durch einen bei atrialer Kardioversion freigesetzten Thrombus, zu minimieren, kann der Arzt die Abgabe eines Kardioversionsschocks mit einem Programmierbaren Parameter bei Patienten ohne hinreichende antithrombotische Medikation verbieten, wenn vor dem Kardioversionsversuch ein ununterbrochenes atriales Flimmern bestand. Diese Funktion ist bereits bekannt.

## Patentansprüche

1. Implantierbarer Herzstimulator mit:
- einer Kardioversions-/Defibrillationseinheit, die mit mindestens einem Elektrodenpaar zur Defibrillations-/Kardioversionsschocktherapie verbunden oder zu verbinden und ausgebildet ist, Kardioversionsschocks oder Defibrillationsschocks zu generieren und abzugeben,
- einer atrialen Sensingeinheit, die ausgebildet ist, eine Vorhofkontraktion zu erfassen und im Falle einer detektierten Vorhofkontraktion ein eine jeweiliges atriales Ereignis anzeigendes atriales Sensingsignal auszugeben,
- einer ventrikulären Sensingeinheit, die ausgebildet ist, eine jeweilige Kammerkontraktion zu erfassen und im Falle einer detektierten Kammerkontraktion ein ventrikuläres Sensingsignal auszugeben,
- einer Tachykardieerfassungseinheit, die mit der atrialen und der ventrikulären Sensingeinheit verbunden und ausgebildet ist, eine Tachykardie zu erfassen und als ventrikuläre Tachykardie (VT) oder als superventrikuläre Tachykardie (SVT) zu klassifizieren, und
- einer Therapiesteuereinheit, die zum Auslösen eines atrialen Kardioversionsschocks mit der Kardioversions-/Defibrillationseinheit verbunden und ausgebildet ist, mindestens einen atrialen Kardioversionsschock auszulösen, wenn zunächst ein von der ventrikulären Sensingeinheit erfasster ventrikulärer Rhythmus schneller als eine programmierte Frequenzgrenze ist und gleichzeitig Tachykardieerfassungseinheit eine SVT als Vorhofflimmern (AFib) klassifiziert.

2. Implantierbarer Herzstimulator nach Anspruch 1, **dadurch gekennzeichnet, dass** die Steuereinheit ausgebildet ist, eine atriale Kardioversion immer nur dann auszulösen, wenn der ventrikuläre Rhythmus schneller als eine programmierbare Frequenzgrenze, jedoch langsamer als der atriale Rhythmus ist und der ventrikuläre Rhythmus unregelmäßig ist.

3. Implantierbarer Herzstimulator nach Anspruch 1 oder 2, **dadurch gekennzeichnet, dass** die Steuereinheit ausgebildet ist, eine atriale Kardioversion immer auf ein von der ventrikulären Sensingeinheit detektiertes intrinsisches Ereignis im Ventrikel zu synchronisieren.

4. Implantierbarer Herzstimulator nach einem der Ansprüche 1 bis 3, **dadurch gekennzeichnet, dass** die Kardioversions-/Defibrillationseinheit mit mindestens 3 Schockelektroden, nämlich einer distalen Schockwendel, einer proximalen Schockwendel und einem elektrisch leitenden Gehäuse des implantierbaren Herzstimulators verbunden oder zu verbinden ist.

5. Implantierbarer Herzstimulator nach einem der Ansprüche 1 bis 4, **dadurch gekennzeichnet, dass** die Kardioversions-/Defibrillationseinheit in Verbindung mit der Therapiesteuereinheit derart ausgebildet ist, dass die Kardioversions-/Defibrillationseinheit für eine atriale Kardioversion und für eine ventrikuläre Defibrillation mit jeweils wenigstens zwei Schockelektroiden verbunden ist, von den wenigstens eine Schockelektrode für die atriale Kardioversion von den Schockelektroden für die ventrikuläre Defibrillation verschieden ist, sodass die Therapiesteuereinheit für die atriale Kardioversion einen anderen Schockpfad einstellen kann als für die ventrikuläre Defibrillation.

6. Implantierbarer Herzstimulator nach einem der Ansprüche 1 bis 5, **dadurch gekennzeichnet, dass** der implantierbare Herzstimulator ein biventrikulärer Dreikammer Herzschrittmacher ist, der mit einer atrialen und zwei ventrikulären Elektrodenleitungen verbunden oder zu verbinden ist, nämlich einer rechtsatrialen Elektrodenleitung, einer rechtsventrikulären Elektrodenleitung und einer linksventrikulären Elektrodenleitung, und der eine linksventrikuläre Sensingeinheit aufweist, die mit einer Sensingelektrode an der linksventrikulären Elektrodenleitung verbunden oder zu verbinden ist und ausgebildet ist, linksventrikuläre intrinsische Ereignisse zu erfassen, wobei die Therapiesteuereinheit vorzugsweise mit der atrialen und der linksventrikulären Sensingeinheit verbunden ist.

7. Implantierbarer Herzstimulator nach Anspruch 6, **dadurch gekennzeichnet, dass** die Steuereinheit ausgebildet ist, eine atriale Kardioversion immer auf ein von der linksventrikulären Sensingeinheit detektiertes intrinsisches linksventrikuläres Ereignis zu synchronisieren.

8. Implantierbarer Herzstimulator nach einem der Ansprüche 1 bis 7, **dadurch gekennzeichnet, dass** der implantierbare Herzstimulator einen Zeitgeber aufweist, der mit der atrialen Sensingeinheit verbunden ist und ausgebildet ist, zu überwachen, ob ein Vorhofflimmern ununterbrochen eine vorgegebene Zeitdauer übersteigt, wobei die Therapiesteuereinheit ausgebildet ist, eine atriale Kardioversion im Falle des Überschreitens der vorgegebenen Zeitdauer zu sperren.

9. Implantierbarer Herzstimulator nach einem der Ansprüche 1 bis 7, **dadurch gekennzeichnet, dass** die Therapiesteuereinheit derart programmierbar ausgebildet ist, dass die Auslösung einer automatischen atrialen Kardioversion durch einen programmierbaren Parameter ein- bzw. ausgeschaltet werden kann.

10. Implantierbarer Herzstimulator nach Anspruch 9, **dadurch gekennzeichnet, dass** der implantierbare Herzstimulator mit einer Telemetrieeinheit zur bidirektionalen, drahtlosen Datenübertragung und mit einem Speicher für Programmparameter ausgestattet ist, so dass der implantierbare Herzstimulator von außen fernprogrammierbar ist.
